# EUROPEAN PATENT APPLICATION

(11) **EP 2 052 734 A1**
(43) Date of publication of application: **29.04.2009**
(21) Application number: 07119403.9
(22) Date of filing: 26.10.2007
(51) Int. Cl.: A61K 38/00, A61K 38/01, A61K 38/16, A61K 38/17, A61P 3/04

(54) **Proteins that stimulate the secretion of satiety hormones**

(71) Applicant: Universiteit Maastricht, 6211 LK Maastricht (NL)
(72) Inventor: The designation of the inventor has not yet been filed
(74) Representative: Habets, Winand

(57) **Abstract**

The invention is in the field of weight management, in particular in the field of weight management by influencing the mechanisms of body-weight regulation. It was found that inhibition of trypsin or DPP IV stimulates secretion of CCK and GLP-1. Several dietary proteins also stimulate the secretion of these hormones. We conclude that the proteins wheat, codfish, egg, egg hydrolysate, and sodium caseinate may be used as dietary ingredients for treatment of overweight and obesity, due to their strong positive effects on satiety hormone release.

## Description

The invention is in the field of weight management, in particular in the field of weight management by influencing the mechanisms of body-weight regulation.

Obesity is one of the major biomedical problems of the last few decades. It is important to find a treatment that affects the mechanisms of body-weight regulation. Ingestion of a high-protein diet induces a significant rise in circulating levels of the gut hormones cholecystokinin (CCK) and glucagon-like peptide 1 (GLP-1) in the blood. This indicates that proteins play an important role in the secretion of these hormones. However, it is unknown which proteins affect secretion of CCK and GLP-1.

The role of proteins in the regulation of long-term energy balance and maintenance of healthy body weight in humans has received little attention. There is some evidence that proteins play an important role in the regulation of food intake and body weight maintenance.

The effects of several proteins on food intake and subjective ratings of hunger and fullness and on gastrointestinal hormone responses like GLP-1 were investigated previously. Energy intake from a buffet meal ad libitum was significantly less after a whey-containing preload, and plasma levels of GLP-1 increased by 60-65% following the whey preload compared with an equivalent casein preload. The whey test meal induced an increase in satiety, which implicates that GLP-1 may serve as potential mediator of the increased satiety response to whey.

GLP-1 acts on stimulation of glucose-dependent insulin secretion and insulin biosynthesis, inhibition of glucagon secretion and gastric emptying, and inhibition of food intake (5). GLP-1 is released in response to nutrient ingestion from endocrine cells distributed throughout the small and large intestine. Following an initial nutrient-stimulated rise in circulating levels of GLP-1, the levels fall rapidly, largely due to renal clearance and the N-terminal degradation of the peptide by dipeptidyl peptidase IV (DPP IV/CD26; EC 3.4.14.5).

DPP IV is a 110 kDa plasma membrane glycoprotein ectopeptidase that belongs to the serine protease family. In mammals, DPP IV is ubiquitously expressed on the surface of endothelial and epithelial cells and highest levels in humans have been reported to occur in the intestine, bone marrow, and kidney. The enzymatic action of DPP IV is important for the brake down of protein hormones and has the capacity to inactivate or modulate gastric inhibitory peptide, glucagon-like peptide (GLP) 1 and 2, and neuropeptide Y, among others. DPP IV in the intestinal brush-border cleaves GLP-1 to an inactive form, and emerging evidence has supported the hypothesis that DPP IV is implicated in the regulation of glucose serum levels ad the control of appetite and satiety.

A semi high throughput system for gut hormone secretion was used to study the role of dietary proteins (ovomucoid, soybean, egg, egg hydrolyate, wheat hydrolysate, whey, pea, casein hydrolysate, sodium caseinate, codfish, and wheat) in hormone secretion. The effects of the dietary proteins on CCK and GLP-1 secretion by STC-1 cells, a murine intestinal cell line, were investigated. STC-1 cells were incubated with a 1% protein solution in HBSS buffer for 2 hours. The supernatant was collected, and CCK and GLP-1 concentrations were determined using RIA assays. All analyses were performed in duplicate.

Effects of the different dietary proteins on GLP-1 and CCK release were compared with the negative control situation (only HBSS buffer). Codfish, egg protein, wheat protein, sodium caseinate, and pea protein induced an increase in GLP-1 release (38301 pM, 96207 pM, 5306, pM, 3705 pM, and 1191 pM respectively) compared to negative control (12,6 pM). CCK levels were increased after addition of wheat protein, codfish protein, and sodium caseinate (51,5 pM, 44,7 pM, and 43,8 pM respectively) compared to the negative control (20,5 pM). All other tested proteins showed no difference in CCK or GLP-1 secretion levels.

Both wheat protein and sodium caseinate induced a large increase in CCK and GLP-1 secretion, whereas pea protein resulted only in increased GLP-1 levels and codfish protein resulted in increased CCK levels. Therefore we conclude that wheat protein, sodium caseinate, pea protein, and codfish protein may be used as dietary ingredients for treatment of obesity.

Serine proteases such as trypsin and dipeptidyl peptidase IV (DPP IV) have been shown to be involved in the regulation of the release and activation of CCK and GLP-1. We found that certain dietary proteins are able to inhibit the activity of serine proteases, and thereby stimulate the secretion of CCK and GLP-1.

Eleven naturally occurring dietary proteins (casein hydrolysate, codfish, egg, egg hydrolysate, ovomucoid, pea, sodium caseinate, soybean, wheat, wheat hydrolysate, and whey) were tested on their effects on satiety hormone secretion. Direct effects of the proteins were determined by incubating STC-1 cells with a 1% protein solution. Indirect effects of the proteins on satiety hormone secretion were investigated by incubating STC-1 cells with either protein and human trypsin or DPP IV. CCK and GLP-1 levels were determined in the supernatant.

Egg hydrolysate, pea, sodium casein, and wheat stimulated CCK release, whereas codfish, egg, egg hydrolysate, sodium caseinate, wheat, and whey stimulated release of GLP-1. All other tested proteins showed no direct effect on the release of CCK and GLP-1.

Exposure of STC-1 cells to a combination of trypsin and either casein hydrolysate, codfish, egg, egg hydrolysate, sodium casein, wheat hydrolysate, or wheat resulted in an additional stimulation of CCK release, compared to the exposure to only the protein. Trypsin in combination with ovomucoid or pea inhibited CCK release. Egg hydrolysate, ovomucoid, or pea in combination with trypsin resulted in decreased levels of GLP-1. Addition of DPP IV in combination with casein hydrolysate, codfish, and wheat hydrolysate resulted in elevated CCK levels, whereas CCK release was inhibited after incubation with DPP IV in combination with egg hydrolysate, ovomucoid, and sodium caseinate. GLP-1 levels were decreased after addition of egg hydrolysate, ovomucoid, and pea in combination with DPP IV. All other tested proteins showed no difference in hormone secretion.

It was found that inhibition of trypsin or DPP IV stimulates secretion of CCK and GLP-1. Several dietary proteins also stimulate the secretion of these hormones. We conclude that the proteins wheat, codfish, egg, egg hydrolysate, and sodium casein may be used as dietary ingredients for treatment of overweight and obesity, due to their strong positive effects on satiety hormone release.

### DETAILED DESCRIPTION OF THE INVENTION

The present study is the first to show that specific dietary proteins are able to influence satiety. DPP IV activity is, however, no direct marker for the effects of the proteins on GLP-1 levels. Pea protein is a strong inhibitor of human DPP IV, but the protein itself showed no effect on GLP-1 release compared to the negative control after 2 hours. Addition of the protein in combination with DPP IV resulted in a dramatic decrease of GLP-1 release. Another remarkable finding is that soybean, a well-known serine protease inhibitor, is not able to inhibit DPP IV activity. Another protein, egg protein hydrolysate, is able to inhibit DPP IV activity, and is able to stimulate GLP-1 release in STC-1 cells, but the combination of egg protein and DPP IV to the cells showed a decrease in GLP-1 levels. This might be due to the inhibitory capacity of the protein. Egg protein hydrolysate and pea protein might inhibit DPP IV in a competitive manner, but the enzyme favours GLP-1 prior to the protein.

Although the inhibition of DPP IV show beneficial effects on health, only chemically synthesised inhibitors are known, and are not commercially available. Mclntosh et al.(Mclntosh CH, Demuth HU, Pospisilik JA, Pederson R. Dipeptidyl peptidase IV inhibitors: how do they work as new antidiabetic agents? Regul Pept 2005; 128: 159-165.) found that oral treatment of DPP IV inhibitor over a 12-week period in DM2 rats had no effect on water or nutrient ingestion, but body weight was decreased by 12.5%. Inhibitor treated diabetic animals showed a marked improvement in glucose tolerance and increased insulin secretion.

DPP IV may be inhibited in several different ways. Competitive, non-competitive, mixed-type, and irreversible inhibition can occur (Lorey S, Stockel-Maschek A, Faust J, Brandt W, Stiebitz B, Gorrell MD, et al. Different modes of dipeptidyl peptidase IV (CD26) inhibition by oligopeptides derived from the N-terminus of HIV-1 Tat indicate at least two inhibitor binding sites. Eur J Biochem 2003; 270: 2147-2156). In most DPP IV inhibition studies, chemically synthesized compounds are used, which inhibit DPP IV either irreversible or in a mixed-type (Bauvois B. A collagen-binding glycoprotein on the surface of mouse fibroblasts is identified as dipeptidyl peptidase IV. Biochem J 1988; 252: 723-731, Farriol M, Pita AM, Fernandez-Bustos MA, Delgado G. Dipeptidyl-peptidase IV in patients with short bowel syndrome. Clin Nutr 2005; 24: 1099-1104, Lugari R, Dei Cas A, Ugolotti D, Barilli AL, Camellini C, Ganzerla GC, et al. Glucagon-like peptide 1 (GLP-1) secretion and plasma dipeptidyl peptidase IV (DPP-IV) activity in morbidly obese patients undergoing biliopancreatic diversion. Horm Metab Res 2004; 36: 111-115). The present study is the first to show that naturally occurring dietary proteins are able to inhibit the activity of human DPP IV.

Pea protein significantly inhibited DPP IV enzyme activity. Only 10% of its activity remained. Other proteins that were able to significantly inhibit the enzyme activity were ovomucoid, whey protein, egg protein, and egg protein hydrolysate. Soybean did not show a decrease in the activity of DPP IV.

Levels of the bioactive form of the satiety hormone GLP-1 present in the blood circulation fall rapidly, due to the degradation of the peptide by DPP IV. GLP-1 plays an important role in the ileal brake, resulting in satiety and weight reduction. Previous studies suggest that GLP-1 secretion is reduced in obese subjects and that weight loss normalizes the levels. The anorectic effects of GLP-1 are, however, preserved in obesity (Stanley S, Wynne K, Bloom S. Gastrointestinal satiety signals III. Glucagon-like peptide 1, oxyntomodulin, peptide YY, and pancreatic polypeptide. Am J Physiol Gastrointest Liver Physiol 2004; 286: G693-697.).

Addition of codfish protein, egg protein, egg protein hydrolysate, sodium caseinate, and wheat protein increased GLP-1 release in STC-1 cells, but in combination with DPP IV, egg protein hydrolysate was not to inhibit the enzyme activity, and the release of GLP-1 decreased significantly. There where no increased levels of GLP-1 observed after addition of the combination of proteins with DPP IV. This is due to the use of a "total GLP-1 RIA". It is not known whether the cells are able to activate the secreted GLP-1, therefore the total GLP-1 RIA was chosen.

We found that adding codfish, egg protein, sodium caseinate, wheat protein, or a combination of these proteins to the diet induced a decrease of DPP IV activity. This will delay GLP-1 degradation, which causes GLP-1 levels to remain elevated during and after a meal containing any of these proteins and result in increased satiety sensations during and after a meal. The increased satiety sensations will decrease the amount of food intake, an on the long term, weight reduction may be induced. Inhibition of DPP IV by dietary proteins may also improve the glucose tolerance and insulin secretion in diabetes mellitus type 2 patients.

### LEGENDS TO THE FIGURES

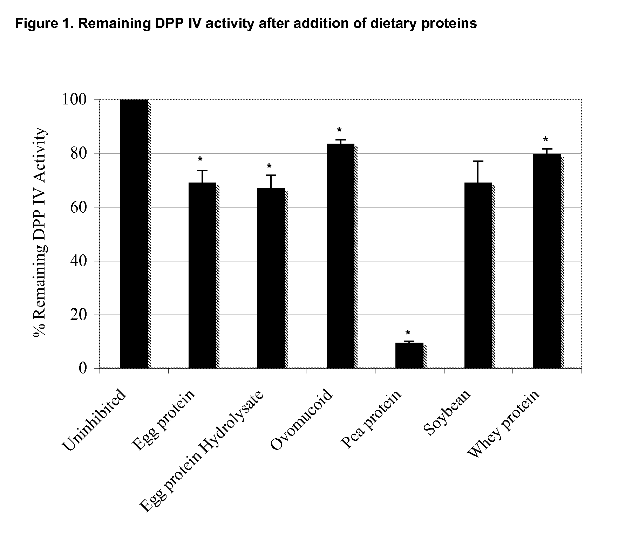

Figure 1: Addition of dietary proteins DPP IV results in decreased activity of the enzyme. Ovomucoid, pea protein, egg protein, egg protein hydrolysate, and whey protein inhibited DPP IV activity, with a remaining activity of 83.5% ± 1.5, 9.6% ± 0.5, and 67.0% ± 4.8 respectively. Results are presented as mean ± SEM.

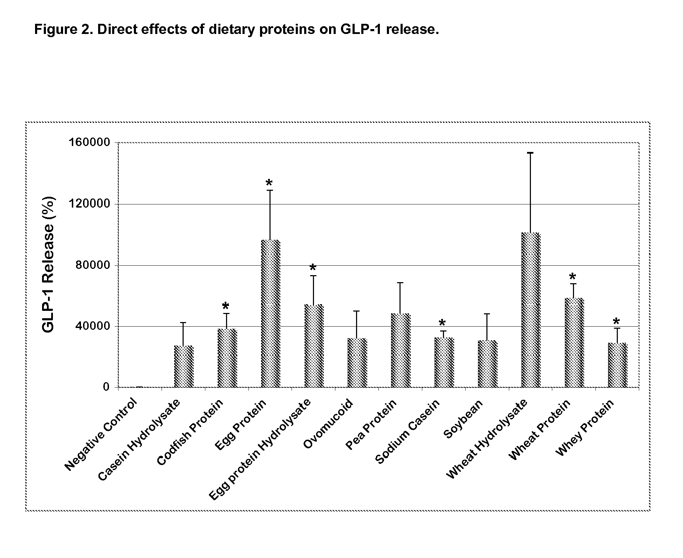

Figure 2: The levels GLP-1 were measured in the supernatant after an exposure time of 2h. Results are expressed as a percentage of the negative control value and represent mean ± SEM of 4 individual experiments. Addition of codfish protein, egg protein, egg protein hydrolysate, sodium caseinate, wheat protein, and whey protein resulted in an increase of GLP-1 release.

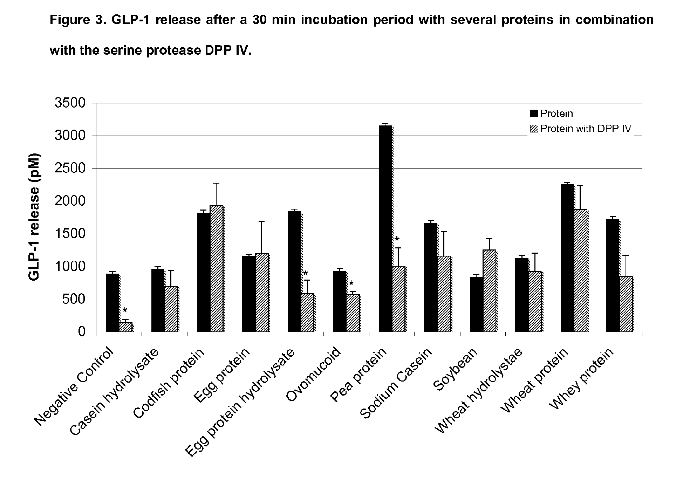

Figure 3: Addition of DPP IV to the negative control resulted in a decreased release of GLP-1. Egg protein hydrolysate, ovomucoid, and pea protein in combination with DPP IV inhibit the secretion of GLP-1. All other proteins show no effect on GLP-1 release in combination with DPP IV.

### EXAMPLES

### Example 1: Materials.

DPP IV (human placenta) and DPP aminopeptidase IV substrate hydrochloride were obtained from MP Biomedicals (Uden, the Netherlands). Ovomucoid (from chicken) and soybean were obtained from Worthington Biochemicals (Huissen, the Netherlands). Egg protein hydrolysate, egg protein, wheat hydrolysate, wheat protein, whey protein, pea protein, casein hydrolysate, sodium caseinate, and codfish protein were kindly provided by DSM Food Specialties (Delft, the Netherlands). The cell line used in the study was the STC-1 cell line. This cell line is derived from an endocrine tumor that developed in the small intestine of a double transgenic mouse expressing the rat insulin promotor linked to the simian virus 40 large T antigen and the polyoma virus small t antigen. STC-1 cells (passage 24) were kindly provided by Dr. Douglas Hanahan (University of California, San Francisco). Other reagents used in this study were purchased from Sigma Aldrich unless indicated differently

### Example 2: Measurement of DPP IV Activity.

The DPP IV activity was measured as described by Bauvois (Bauvois B. A collagen-binding glycoprotein on the surface of mouse fibroblasts is identified as dipeptidyl peptidase IV. Biochem J 1988; 252: 723-731.) with some modifications described by Farriol et al. (Farriol M, Pita AM, Fernandez-Bustos MA, Delgado G. Dipeptidyl-peptidase IV in patients with short bowel syndrome. Clin Nutr 2005; 24: 1099-1104.). Briefly, a solution of dehydrated trisodic citrate (10 mM in saline solution pH 6.0) was used as buffer. The enzymatic substrate was DPP aminopeptidase IV substrate hydrochloride (1.11 mM in distilled water). The enzyme assay was performed in a cuvette containing a final volume of 1 ml: 250 µl buffer, 300 µl sample and 450 µpl substrate. The reaction mix was monitored before and after an incubation period of 60 min at 37 °C at a wavelength of 450 nm against a negative control. Enzyme and inhibitor were preincubated for 30 min at 37°C. Remaining enzyme activity was measured by adding substrate to a final concentration of 0.5 mM and the reaction was continued for 1 h. Control mixtures lacking enzyme as negative control or inhibitor as positive control were also tested. Remaining activity is expressed as percentage of the control activity (without inhibitor).

### Example 3: Cell culture conditions.

STC-1 cells (passage 25 to 40) were maintained in Dulbecco's Modified Eagles Medium (DMEM; Invitrogen) with 10% fecal bovine serum (FBS; Invitrogen), 2 mM L-glutamine, 100 units/ml penicillin, and 100 µg/ml streptomycin as additional supplements; at 37°C in 5% CO2/air.

### Example 4: Secretion studies.

Two sets of secretion assays were performed. The first assays were performed to study the direct effects of dietary proteins on the secretion of several satiety hormones. Briefly, tree days before the experiment, STC-1 cells were seeded in 24-well plates (1.0 x 105 cells/well). On the day of the experiment, cells were first washed 2 times with PBS, followed by addition of a 1% protein-solution to each well. After an incubation period of 2 hours at 37°C, the supernatant was used to measure secreted GLP-1.

In the second set, the indirect effects of the dietary proteins were tested on secretion of satiety hormones. Briefly, on the day of experiment, cells were incubated with a mix of a 1% protein solution with human trypsin (Athens Research; Georgia, USA) or human DPP IV (Athens Research; Georgia, USA), and incubated at 37°C for 30 min. The supernatant was tested for secreted GLP-1.

### Example 5: Measurement of GLP-1.

GLP-1 levels were determined using RIA (GLP1T-36HK, Linco Research, Missouri, USA). The detection level of this kit is 3 to 333 pM. The intra-assay variation ranges from 10 to 23% and the inter-assay variation from 22 to 38%. There is no cross-reaction with GLP-2 and glucagon (0.01% and 0.2%, respectively).

### Example 6: Statistical analysis.

The descriptive and statistical analyses were performed with SPSS, version 11.0. The means of the variables are presented with their standard deviation (mean ± SD). Comparison of means of the inhibition studies was done using a one-sample t-test. Comparison of means of the secretion studies was done using the one-way ANOVA. A P-value of less than 0.05 was considered statistically significant.

### Example 7: Inhibition of DPP IV by dietary proteins

Only six proteins (egg protein, egg protein hydrolysate, ovomucoid, pea protein, soybean, and whey protein) were tested for their inhibitory effects on the activity of DPP IV. Ovomucoid, pea protein, egg protein, egg protein hydrolysate, and whey protein inhibited DPP IV activity, with a remaining activity of 83.5% ± 1.5, 9.6% ± 0.5, and 67.0% ± 4.8 respectively (figure 1).

### Example 8: Direct effect of proteins on GLP-1 secretion

Addition of a 1% protein solution to STC-1 cells resulted in increased levels of GLP-1 in the supernatant compared to the negative control (only HBSS buffer; 100%). Codfish protein, egg protein, egg protein hydrolysate, sodium caseinate, wheat protein, and whey protein were able to significantly elevate the release of GLP-1 into the supernatant (38302% ± 10181, 96207% ± 32670, 54120% ± 19112, 32521% ± 4524, 58259% ± 9460, 28879% ± 9806; respectively) (figure 2).

### Example 9: Effects of DPP IV in combination with dietary proteins in GLP-1 secretion

Addition of DPP IV to the negative control resulted in a decreased release of GLP-1 (886 pM to 143 pM± 46). Decrease levels of GLP-1 were also observed after combining DPP IV with egg protein hydrolysate (1838 pM to 584 pM ± 207), ovomucoid (930 pM to 567 pM ± 56), and pea protein (3149 pM to 1002 pM ± 285). All other proteins show no effect on GLP-1 release in combination with DPP IV (figure 3).

## Claims

1. Use of a protein preparation selected from the group consisting of wheat protein, sodium caseinate, pea protein, egg protein, egg hydrolysate and codfish protein for inducing satiety in a human being or in an animal.

2. Use of a protein preparation selected from the group consisting of wheat protein, sodium caseinate, pea protein, egg protein, egg hydrolysate and codfish protein for the preparation of a medicament for the treatment of obesity.

3. Use of a food composition comprising a protein preparation selected from the group consisting of wheat protein, sodium caseinate, pea protein, egg protein, egg hydrolysate and codfish protein for inducing satiety in a human being or in an animal.
